(19) Europäisches Patentamt
European Patent Office
Office européen des brevets

(11) EP 1 535 603 B1

(12) # EUROPÄISCHE PATENTSCHRIFT

(45) Veröffentlichungstag und Bekanntmachung des Hinweises auf die Patenterteilung:
**17.05.2017 Patentblatt 2017/20**

(51) Int Cl.:
***A61K 8/44*** *(2006.01)* ***A61Q 17/00*** *(2006.01)*
***A61Q 19/00*** *(2006.01)* ***A61Q 19/08*** *(2006.01)*
***A61K 8/19*** *(2006.01)* ***A61K 31/198*** *(2006.01)*

(21) Anmeldenummer: **04026787.4**

(22) Anmeldetag: **11.11.2004**

(54) **Kosmetische und dermatologische Zubereitung, enthaltend Kreatin und/oder Kreatinin und Elektrolytkonzentrationen mit einer Ionenstärke von mindestens 50 mmol/l**

Cosmetic or dermatological preparation containing creatin and/or creatinin and concentrations of electrolytes of at least 50 mmol/l

Préparations cosmétique et dermatologique contenant de la créatine et/ou de la créatinine et des concentrations d'électrolytes d'au moins 50 mmol/l.

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **26.11.2003 DE 10355711**

(43) Veröffentlichungstag der Anmeldung:
**01.06.2005 Patentblatt 2005/22**

(73) Patentinhaber: **Beiersdorf AG**
**20253 Hamburg (DE)**

(72) Erfinder:
- **Kallmayer, Volker, Dr.**
**22525 Hamburg (DE)**
- **Mummert, Christopher, Dr.**
**29553 Bienenbüttel (DE)**
- **Raschke, Thomas, Dr.**
**25421 Pinneberg (DE)**

(56) Entgegenhaltungen:
**WO-A-02/02075**
**WO-A-97/45026**
**WO-A-02/076408**
**WO-A-03/005988**
**WO-A-03/011241**
**WO-A-2004/064801**
**US-A- 5 908 864**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 535 603 B1

## Beschreibung

Die vorliegende Erfindung betrifft kosmetische Zubereitungen mit einem Gehalt an Kreatin und/oder Kreatinderivaten in kosmetischen oder dermatologischen Zubereitungen zur Behandlung und Prophylaxe der Symptome von UV- und oder Ozon- induzierten Hautschäden sowie von entzündlichen und degenerativen Hautzuständen.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z.B. nach dem Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A (Retinol) und/oder dessen Derivate. Ihre Wirkung auf die Strukturschäden ist allerdings nur von begrenztem Umfang. Darüber hinaus gibt es bei der Produktentwicklung erhebliche Schwierigkeiten, die Wirkstoffe ausreichend gegen oxidativen Zerfall zu stabilisieren. Die Verwendung Vitamin A-Säurehaltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen

Reaktionsprodukte in den Hautmetabolismus eingreifen.

Vorwiegend handelt es sich bei solchen photochemischen Reaktionsprodukten um radikalische Verbindungen, beispielsweise Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalisches Sauerstoffmolekül im angeregten Zustand kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Antioxidantien werden hauptsächlich als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Kalifornien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Die vorteilhafte prophylaktische und therapeutische Wirkung des Kreatins bei der kosmetischen und medizinischen Hautpflege ist an sich bekannt. Kreatin (von grch.: το κρεαζ = "das Fleisch") zeichnet sich durch folgende Struktur aus:

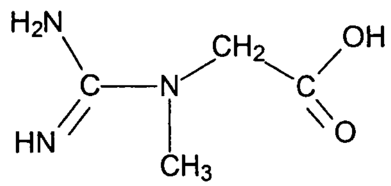

Es findet sich im Muskelgewebe der Wirbeltiere zu 0,05-0,4%, in geringen Mengen auch im Gehirn und Blut. Als Monohydrat stellt es ein farbloses, kristallines Pulver dar. In wässriger Lösung wird durch eine intramolekulare Cyclisierung Kreatinin gebildet. Im Organismus entsteht es durch Transamidinierung von L-Arginin auf Glycin zu Guanidinoessigsäure und deren anschließende Methylierung mittels S-Adenosylmethionin (durch Guanidinoacetat-Methyltransferase). Man hält Kreatin für einen appetitfördernden Bestandteil von Rindfleisch und Fleischextrakt. Kreatinzusatz zur Nahrung verstärkt die körperliche Leistungsfähigkeit.

Kreatinin (ebenfalls von grch.: το κρεαζ = "das Fleisch") ist durch folgende Struktur gekennzeichnet

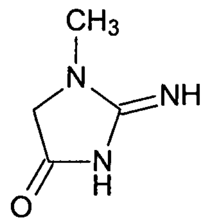

und entsteht im Organismus durch nichtenzymatische Umwandlung aus Kreatinphosphat gemäß

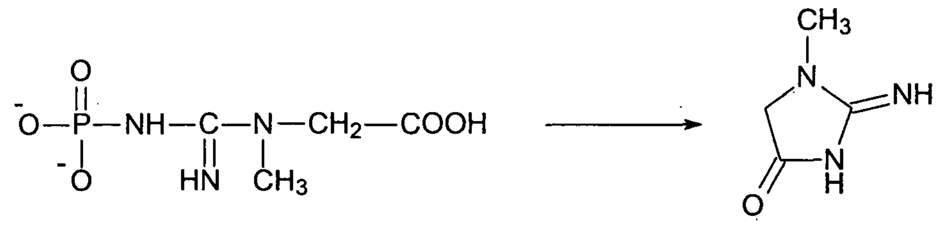

und wird über die Niere ausgeschieden. Die Menge der Kreatininausscheidung ist der Muskelmasse proportional und für das jeweilige Individuum annähernd konstant. Kreatinin ist in Fleischextrakt und Fleischbrühwürfeln enthalten.

Umfangreich ist der Stand der Technik zu den kosmetischen und dermatologischen Verwendungen des Kreatins.

So beschreibt die DE 100 32 964 die Verwendung von Kreatin und/oder Kreatinderivaten in kosmetischen oder dermatologischen Zubereitungen zur Behandlung und Prophylaxe der Symptome von UV- und oder Ozon- induzierten Hautschäden sowie von entzündlichen und degenerativen Hautzuständen.

Die JP2000/247866 beschreibt Hautkosmetika mit einem Gehalt an Kreatin und/oder Kreatinin, welche als Creme oder als milchige Lotion verwendet werden können, wobei den betreffenden Zubereitungen vorzügliche hautpflegende Eigenschaften zugeschrieben werden.

Ferner beschreibt die WO00/33787 die Verwendung von Kreatinin als wirksamen Bestandteil von Desodorantien.

Weiterhin beschreibt die EP-A 565 010 Haarwuchs- und Haarfärbezubereitungen mit einem Gehalt an Kreatininphosphat.

Schließlich werden in der US-A 4,590,067 und der EP-A-178 602 die Verwendung von Kreatin bzw. Kreatinin zur Herstellung von Zubereitungen mit antünflammatorischer Wirksamkeit beschrieben.

Nachteilig ist jedoch, dass Kreatin in wasserhaltigen Produkten leicht auskristallisiert, wobei Kristalle mit unkosmetischer Anmutung entstehen und die Wirksamkeit des Produktes vermindert wird.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Eine weitere Aufgabe bestand darin, eine Darreichungsform für Kreatin zu finden, die sich durch verminderte Neigung zur Bildung von Kreatinkristallen auszeichnet.

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es zweckmäßiger für die Schilderung der vorliegenden Erfindung und ihres technischen Hintergrundes, von der Ionenstärke eines gegebenen Elektrolyts in seiner Lösung auszugehen. Die Ionenstärke *I* einer Elektrolytlösung ist definiert als

$$I = \frac{1}{2} \sum_i c_i \, z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige ( = 0,17-molare) wässrige Kochsalzlösung hat beispielsweise eine Ionenstärke *I*= 0,17 mol / l.

Eine weitere Aufgabe der vorliegenden Erfindung war es also, Lösungswege zu kosmetischen oder dermatologischen Emulsionen, insbesondere O/W- oder W/O-Emulsionen, aufzudecken, welche gegenüber erhöhten Elektrolytkonzentrationen - bzw. erhöhten lonenstärken - stabil sind.

Ferner war eine Aufgabe der vorliegenden Erfindung, Zubereitungen zur Verfügung zu stellen, welche den Zustand der Haut deutlich verbessern, insbesondere die Hautrauigkeit vermindern.

Es hat sich überraschend gezeigt, und darin liegt die Lösung dieser Aufgaben, dass eine Kosmetische Zubereitung mit einer wässrigen Phase, enthaltend

(a) Kreatin und/oder Kreatinin
(b) wobei in der der wässrige Phase ein oder mehrere Elektrolyte

gewählt aus der Gruppe

(1) der Salze mit folgenden Anionen: Chloride, ferner anorganische Oxo-Element-Anionen, von diesen insbesondere Sulfate, Carbonate, Phosphate, Borate und Aluminate, auf organischen Anionen basierende Elektrolyte, z.B. Lactate, Acetate, Benzoate, Propionate, Tartrate, Citrate, Aminosäuren, Ethylendiamintetraessigsäure und deren Salze
und folgenden Kationen: Ammonium,- Eisen-, Kupfer-, Zink-, Aluminium-, Alkylammonium,- Alkalimetall-, Erdalkalimetall,- insbesondere Natrium-, Calcium- und Magnesiumionen;
(2) der als Alkalisalze vorliegenden wasserlöslichen UV-Filtersubstanzen, insbesondere solcher, die an ihrem Molekülgerüst eine oder mehrere Sulfonsäuregruppen bzw. Sulfonatgruppen tragen;
(3) der Aminosäuren und deren Salze bzw. deren Anionen;
(4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen

gelöst enthalten sind, wobei die Ionenstärke der wässrigen Phase, in der der oder die Elektrolyte gelöst vorliegen, mindestens 0,050 mol/l beträgt und mindestens diese Wasserphase einen pH-Wert von 6,2 bis 7,8, besonders bevorzugt 6,5 bis 7,5 aufweist.

den Nachteilen des Standes der Technik abhelfen.

Es war für den Fachmann daher nicht vorauszusehen, dass die erfindungsgemäßen Zubereitungen

- besser als feuchtigkeitsspendende Zubereitungen wirken,
- besser die Hautglättung fördern,
- sich durch besser Pflegewirkung auszeichnen,
- besser als Vehikel für kosmetische und medizinisch-dermatologische Wirkstoffe dienen
- höhere Stabilität gegenüber Zerfall in Öl- und Wasserphasen aufweisen und
- sich durch bessere Bioverträglichkeit auszeichnen würden

als die Zubereitungen des Standes der Technik.

Bevorzugt enthalten kosmetische oder dermatologische Zubereitungen gemäß der Erfindung 0,0001 - 10 Gew.-%, besonders bevorzugt 0,001 - 1 Gew.-%, an Kreatinin und/oder Kreatininderivaten, bezogen auf die Gesamtzusammensetzung der Zubereitungen.

Es ist erfindungsgemäß sowohl vorteilhaft, Kreatin ohne die Gegenwart von Kreatinin und Kreatinin ohne die Gegenwart von Kreatin einzusetzen. Besonders vorteilhaft ist allerdings, beide Substanzen gleichzeitig in den erfindungsgemäßen Wirkstoffkombinationen und Zubereitungen zu verwenden, insbesondere, wenn das Gewichtsverhältnis von Kreatinin zu Kreatin aus dem Bereich von 10 : 1 bis 1 : 10, besonders bevorzugt von 4 : 1 bis 3 zu 7, ganz besonders bevorzugt 2 : 1 bis 1 : 2 gewählt wird.

Es ist von Vorteil, die Ionenstärke der erfindungsgemäßen Emulsion so auszugestalten, dass ihr Wert zwischen 0,05 mol / l und 1,00 mol / l, bevorzugt zwischen 0,05 und 0,60 mol / l, insbesondere bevorzugt zwischen 0,05 und 0,4 mol / l liegt.

Die Einstellung der Ionenstärke erfolgt aufgrund einfacher stöchiometrischer Berechnungen, die dem Fachmann bekannt sind. Dabei wird davon ausgegangen, dass alle ionogenen Verbindungen vollständig dissoziiert vorliegen. Es ist zu berücksichtigen, dass bei der Herstellung einer erfindungsgemäßen Zubereitung durch Mischen von Säuren und Laugen Salze gebildet werden können, die formal nicht in einer Rezeptur erscheinen, aber dennoch die Ionenstärke beeinflussen. Auch solche Salze müssen mit einbezogen werden.

Erfingdungsgemäß werden der oder die Elektrolyte ferner vorteilhaft gewählt aus der Gruppe der Z-Phenylbenzimidazol-5-suffonsäure und ihrer Salze, beispielsweise das Natrium-, Kalium- oder ihr Triethänolammonium-Salz

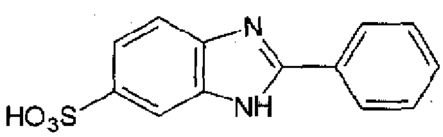

der Sulfonsäure-Derivate von Benzophenonen, vorzugsweise 2-Hydroxy-4-methoxybenzophenon-5-sulfonsäure und deren Salzen, beispielsweise das entsprechende Natrium-, Kalium- oder Triethanolammonium-Satz:

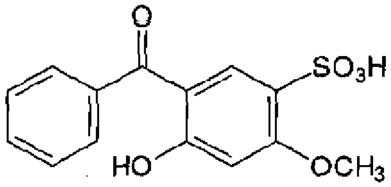

Der Sulfonsäure-Derivate des 3-Benzylidencamphers, wie z.B. 4-(2-Oxo-3-bornylidenmethyl)benzolsulfonsäure und deren Salzen, beispielsweise das entsprechende Natrium-, Kalium- oder Triethanolammonium-Salz:

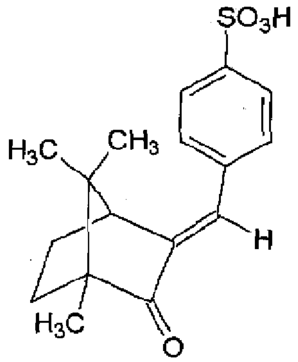

der 2-Methyl-5-(2-oxo-3-bornylidenrnethyl)benzalsulfonsäure und deren Salze, beispielsweise das entsprechende Natrium-, Kalium- oder Triethanolammonium-Salz:

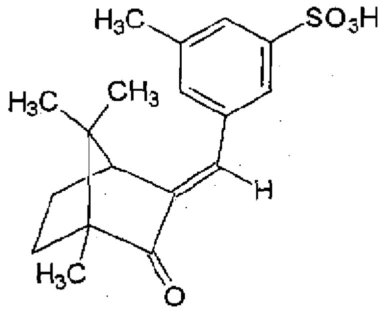

des 1,4-di(2-oxo-10-Sulfo-3-bornylidenmethyl-)Benzols und dessen Salzen (die entsprechenden 10-Sulfato-verbindungen, beispielsweise das entsprechende Natrium-, Kalium- oder Triethanolammonium-Salz), auch als Benzol-1,4-di(2-oxo-3-bornylidenmetbyl-10-Sulfonsäure bezeichnet;

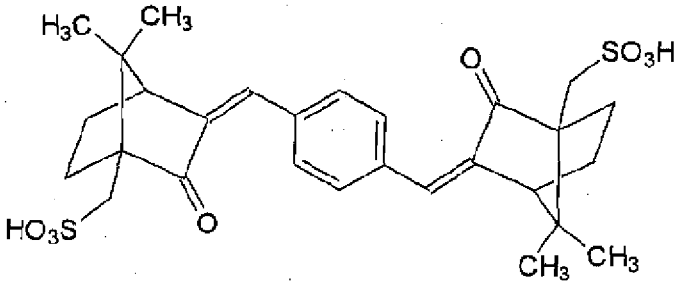

Weitere vorteilhafte UV-A-Filtersubstanzen sind die Phenylen-1,4-bis-(2-benzimidazyl)-3,3'-5,5'-tetrasulfonsäure

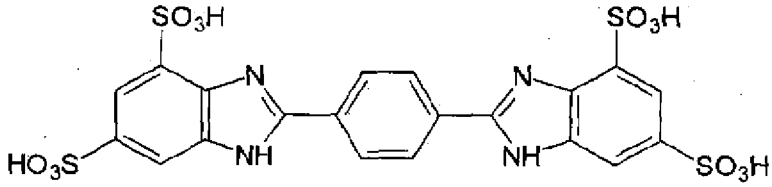

und ihre Salze, besonders die entsprechenden Natrium-, Kalium- oder Triethanolarnmonium-Salze, insbesondere das Phenylen-1,4-bis-(2-benzimidazyl)-3,3'-5,5'-tetrasulfonsäure-bis-natriumsalz

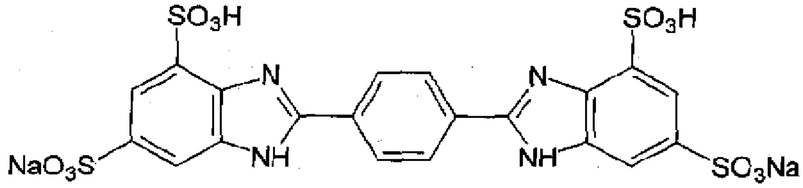

mit der INCI-Bezeichnung Bisimidazylate, welches beispielsweise unter der Handelsbezeichnung Neo Heliopan® AP bei Haarmann & Reimer erhältlich ist.

Erfindungsgemäß werden der oder die Elektrolyte weiterhin vorteilhaft gewählt aus der Gruppe

(3) der Aminosäuren und deren Salze bzw. deren Anionen. Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydratationsvorgänge Feuchtigkeit in der Haut gebunden werden kann.
Aminosäuren mit besonders vorteilhafter kosmetischer bzw. dermatologischer Wirkung sind Glycin, Alanin, Valin, Leucin, Isoleucin; Phenylalanin, Tyrosin, Prolin, Hydroxyprolin, Serin, Threonin, Cystein, Methionin, Tryptophan, Arginin.

Erfindungsgemäß werden der oder die Elektrolyte weiter vorteilhaft gewählt aus der Gruppe

(4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen.
α-Hydroxycarbonsäuren, welche kosmetisch oder dermatologisch relevant sind, folgen der allgemeinen Formel

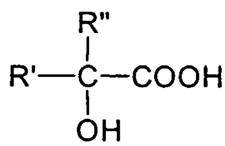

β-Hydroxycarbonsäuren, welche kosmetisch oder dermatologisch relevant sind, folgen der allgemeinen Formel

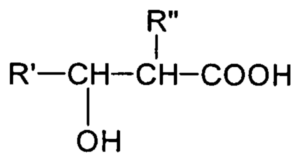

bzw.

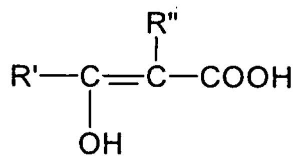

α-Ketocarbonsäuren, welche kosmetisch oder dermatologisch relevant sind, folgen der allgemeinen Formel

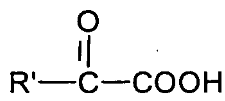

wobei jeweils R’ und R" unabhängig voneinander gewählt werden aus der Gruppe

(a1) H-,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(a4) Phenyl-,
(a5) mit einer oder mehreren Carboxylgruppen und/oder Hydroxygruppen und/oder verzweigten und/oder unverzweigten $C_{1-25}$-Alkylgruppen substituiertes Phenyl-, oder wobei das α-Kohlenstoffatom und das β-Kohlenstoffatom der β-Hydroxycarbonsäure mit R’ und R" zusammen eine
(a6) unsubstituierte Cycloalkylgruppe oder Arylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen
ausbildet und
wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:
Die Salicylsäure (auch 2-Hydroxybenzoesäure, Spirsäure), welche durch die Struktur

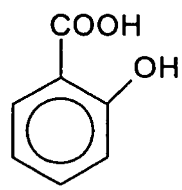

gekennzeichnet ist. Bekanntermaßen wirkt Salizylsäure antibakteriell und keratolytisch und ist Bestandteil mancher kosmetischen oder pharmazeutischen Zubereitungen.
Die den erfindungsgemäß verwendeten α-Hydroxycarbonsäuren werden vorteilhaft gewählt aus folgenden Substanzklassen:

(a2) α-Hydroxyfettsäuren, wobei diese wiederum besonders vorteilhaft aus der Gruppe der $C_{10-18}$-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe

- α-Hydroxycarbonsäuren, gemäß der Formel

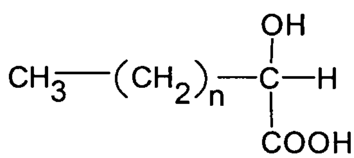

und/oder

- α-Hydroxy-isocarbonsäuren, gemäß der Formel

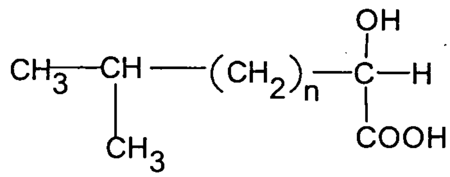

und/oder

- α-Hydroxy-anteisocarbonsäuren, gemäß der Formel

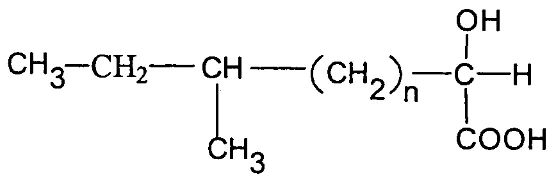

,

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

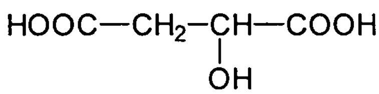

Milchsäure (2-Hydroxypropansäure) ist durch folgende chemische Struktur gekennzeichnet:

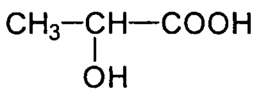

Citronensäure (2-Hydroxy-1,2,3-propantricarbonsäure) ist durch folgende chemische Struktur gekennzeichnet:

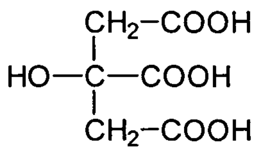

Bekanntermaßen wird Citronensäure zur Pufferung kosmetischer und/oder dermatologischer Zubereitungen,

aber auch als Synergist für Antioxidantien in der Haut- und Haarkosmetik verwendet.

Weinsäure (Dihydroxybernsteinsäure) ist durch folgende chemische Struktur gekennzeichnet:

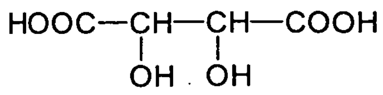

Bevorzugte α-Ketocarbonsäure ist die Brenztraubensäure (α-Oxopropansäure). Sie zeichnet sich durch folgende Struktur aus:

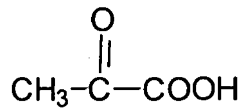

Die Höchstmenge der einzusetzenden Elektrolyte ist letztendlich abhängig von deren Löslichkeit in der wässrigen Phase. Grundsätzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolyts hinausgehende zusätzliche Menge dieses Elektrolyts, beispielsweise als ungelösten Festkörper, in eine kosmetische oder dermatologische Zubereitung einzuarbeiten.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Es ist dem Fachmanne natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Rektion gemäß

$$n\ TiO_2 + m\ (RO)_3\ Si\text{-}R' \text{-}> n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Entsprechend können kosmetische und/oder topische dermatologische Zusammensetzungen im Sinne der vorliegenden Erfindung, je nach ihrem Aufbau, beispielsweise verwendet werden als Hautpflegeprodukt, Hautschutzprodukt, Reinigungsprodukt, Sonnenschutzprodukt, Haarkur, Körperreinigungsprodukt, für die Tages- oder Nachtpflege, die Pflege bestimmter Hautareale wie Hände, Gesicht, Füße usw.

Auch ist die Verwendung der erfindungsgemäßen Zubereitungen zur Prophylaxe und Behandlung der Symptome der lichtgealterten Haut sowie von Altershaut, zur Verhinderung und Verminderung der Entstehung und Ausbreitung von Fältchen und Falten sowie zur Behandlung und Pflege gealterter Haut erfindungsgemäß.

Weiterhin ist die Verwendung der erfindungsgemäßen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rhizinusöl), Ceramide, insbes. Ceramid I, III und VI, Cholesterin, Phytosterole, Fettsäuren mit einer Kettenlänge von C16-26, Carnitin und seine Derivate, Harnstoff, Polyole wie Glycerin, Butylenglykol, Propylenglykol und Dipropylenglykol, Pseudoceramide; Elektrolyte wie Natriumchlorid und Taurin, Fettalkohole sowie Wachse.

Außerdem ist die Verwendung der erfindungsgemäßen Zubereitungen zur Prophylaxe und Behandlung der Symptome der sensiblen bzw. entzündeten Haut vorteilhaft. Bevorzugte Wirkstoffe für diesen Einsatzzweck sind: Hamamelisextrakt, Kamillenextrakt, Glycerrhizinsäure, Acetylsalicylsäure, Diclofenac, Pentacyclische Triterpene (Sericoside, Ursolsäure) und Panthenol.

Außerdem ist die Verwendung der erfindungsgemäßen Zubereitungen zur Prophylaxe und Behandlung der Symptome der fehlpigmentierten Haut vorteilhaft. Bevorzugte Wirkstoffe für diesen Einsatzzweck sind: Tyrosinaseinhibitoren, Hydrochinonderivate, Dioic Acid, Liponsäure, Ascorbinsäure und ihre Derivate sowie Kojisäure.

Nicht zuletzt ist die Verwendung der erfindungsgemäßen kosmetischen und/oder dermatologischen Zubereitungen zur Prophylaxe, Behandlung und Reinigung fettiger Haut sowie zur Prophylaxe und Behandlung von unreiner Haut sowie von Cellulite erfindungsgemäß.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfume, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäu - re) und deren Derivate, Peptide wie D,L-Carnosin, D-Carnosin, L-Car nosin und deren Derivate (z.B. Anserin), Carotinoide, Carotine (z.B. $\alpha$-Carotin, $\beta$-Ca rotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoxi minverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure,

Phytin säure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Fol säure und deren Derivate, Ubichinon und Ubichinol und deren Derivate (insbesondere Ubichinon Q10), Vitamin C und De rivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, pflanzliche Polyphenole mit einem logP von 1-3, α-Glycosylrutin, Ferulasäure, Kaffeesäure, Propylgallat, Furfurylidenglucitol, Carnosin, Butylhydroxytoluol, Butylhydroxy anisol, Nordihydroguajakharzsäure, Nordihydroguajaretsäure, Trihydroxybutyrophenon, Harn säure und deren Derivate, Mannose und deren Derivate, Zink und dessen Derivate (z.B. ZnO, $ZnSO_4$ Selen und dessen Derivate (z.B. Se lenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Besonders vorteilhaft im Sinne der vorliegenden Erfindung können Öl-lösliche Antioxidantien eingesetzt werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Bevorzugte Emulgatoren sind O/W-Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R-O-(-CH_2-CH_2-O-)_n-R'$,
- der Fettsäureethoxylate der allgemeinen Formel

$$R-COO-(-CH_2-CH_2-O-)_n -H,$$

- der veretherten Fettsäureethoxylate der allgemeinen Formel

$$R-COO-(-CH_2-CH_2-O-)_n -R',$$

- der veresterten Fettsäureethoxylate der allgemeinen Formel

$$R-COO-(-CH_2-CH_2-O-)_n -C(O)-R',$$

- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel $R-O-(-CH_2-CH_2-O-)_n-CH_2-COOH$ nd n eine Zahl von 5 bis 30 darstellen,
- der Polyoxyethylensorbitolfettsäureester,
- der Alkylethersulfate der allgemeinen Formel $R-O-(-CH_2-CH_2-O-)_n-SO_3-H$
- der Fettalkoholpropoxylate der allgemeinen Formel

$$R-O-(-CHZ-CH(CH3)-O-)_n-H,$$

- der Polypropylenglycolether der allgemeinen Formel

$$R-O-(-CH_2-CH(CH_3)-O-)_n-R',$$

- der propoxylierten Wollwachsalkohole,
- der veretherten Fettsäurepropoxylate

$$R\text{-}COO\text{-}(\text{-}CH_2\text{-}CH(CH_3)\text{-}O\text{-})_n\text{-}R',$$

- der veresterten Fettsäurepropoxylate der allgemeinen Formel

$$R\text{-}COO\text{-}(\text{-}CH_2\text{-}CH(CH_3)\text{-}O\text{-})_n\text{-}C(O)\text{-}R',$$

- der Fettsäurepropoxylate der allgemeinen Formel

$$R\text{-}COO\text{-}(\text{-}cH_2\text{-}CH(CH_3)\text{-}O\text{-})_n\text{-}H,$$

- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

$$R\text{-}O\text{-}(\text{-}CH_2\text{-}CH(CH_3)O\text{-})_n\text{-}CH_2\text{-}COOH$$

- der Alkylethersulfate bzw. die diesen Sulfaten zugrundeliegenden Säuren der allgemeinen Formel

$$R\text{-}O\text{-}(\text{-}CH_2\text{-}CH(CH_3)\text{-}O\text{-})_n\text{-}SO_3\text{-}H$$

- der Fettalkoholethoxylate/propoxylate der allgemeinen Formel

$$R\text{-}O\text{-}X_n\text{-}Y_m\text{-}H,$$

- der Polypropylenglycolether der allgemeinen Formel

$$R\text{-}O\text{-}X_n\text{-}Y_m\text{-}R',$$

- der veretherten Fettsäurepropoxylate der allgemeinen Formel

$$R\text{-}COO\text{-}X_n\text{-}Y_m\text{-}R',$$

- der Fettsäureethoxylate/propoxylate der allgemeinen Formel

$$R\text{-}COO\text{-}X_n\text{-}Y_m\text{-}H,.$$

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether - (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Poly ethylenglycol(18)isocetylether (Isoceteth-18), Polyethylenglycol(19)isocetylether (Isoceteth-19), Polyethylenglycol(20) isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethy lenglycol-(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen: Polyethylenglycol(20)stearat,- Polyethylenglycol(21 )stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17) isostearat, Polyethylenglycol(18)isostearat, Polyethylenglycol(19)isostearat, Polyethylen glycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Prim rose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21 )glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylen glycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Besonders bevorzugte O/W-Emulgatoren sind Triceteareth-4-Phosphat, Glycerylstearatcitrat, Cethyl-Dimethicone-Copolyol; Wollwachsalkohol, Methylglucosesesquistearat, PEG-PPG-Blockpolymere (Pluronics F68 / 127), Cetearylglucosid, Stearinsäure, PEG-100-Stearat, PEG-40-Stearat, PEG-30-Stearat, PEG-25-Stearat, PEG-20-Glycerylstearat, PEG-25 Hydrogeniertes Ricinusöl, Ceteareth-25, Ceteareth-20, Ceth eareth-12, Steareth-21, Steareth-12, Steareth-2 und Polyglyceryl-3-Methylglucosedistearat.

Dem Fachmanne ist bekannt, dass das Einstellen einer Ionenstärke von mehr als 0,010 mol/l das Vermögen vieler Emulgatoren, Öl in Wasser stabil zu emulgieren, nachteilig beeinflusst. Dies ist insbesondere der Fall bei Emulgatoren, deren Wirkung hauptsächlich auf der Stabilisierung durch elektrostatische Abstoßung basiert, beispielsweise den Alkalisalzen der Fettsäuren.

Vorteilhaft ist also die Verwendung von elektrolytstabilen O/W-Emulgatoren. Als elektrolytstabile O/W-Emulgatoren sind solche zu bezeichnen, die die Emulsionströpfchen auch durch sterische Abstoßung stabilisieren, im Wesentlichen also solche, die eine oder mehrere Polyethylenglykolketten mit mehr als 10 Polyethylenglykol-Einheiten oder eine oder mehrere Polyglycerylketten mit mehr als zwei Glyceryl-Einheiten enthalten

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen, sowie Lanolinalkohol.

Bevorzugte W/O-Emulgatoren sind Polyglyceryl-3 Diisostearat, Polyglyceryl-4 Isostearat, Polyglyceryl-2 Dipolyhydroxystearat, Cetyl PEG/PPG-10-1- Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodecylglycolgopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind Poylethylenglycol-45/Dodecylglycolcopolymer, Polyglyceryl-3-Diisostearat, PEG-30 Dipolyhydroxystearate, Sorbitanisostearate, Sorbitan Stearate, Glyceryl Isostearate, Glyceryl Stearate und Sorbitan Oleate.

Es ist auch möglich, auf das Herabsetzen der Grenzflächenenergie durch Emulgatoren oder Tenside zu verzichten und statt dessen die Grenzfläche durch Anlagerung von in beiden Phasen unlöslichen Partikeln zu stabilisieren. Hierzu können natürliche oder künstliche Polymere (Polyethylen, Nylon, Stärke und ihre Derivate) oder anorganische Partikel ($TiO_2$, $Al_2O_3$, $BaSO_4$, BN, Silikate, Alumosilikate) verwendet werden.

Die Lipidphase kann vorteilhaft gewählt werden aus folgenden Substanzgruppen:

- Mineralöle, Mineralwachse
- Öle, wie Triglyceride der Caprin- oder der Caprylsäure, Rizinusöl, Fettalkoholether, wie Dicaprylylether, oder Ester der Kohlensäure mit Fettalkoholen, wie Dicaprylylcarbonat,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wässrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)-ester;

Vorteilhafte wasserlösliche UVB-Filtersubstanzen sind bereits unter dem Begriff der erfindungsgemäß vorteilhaften Elektrolyte genannt worden, seien hier aber noch einmal auszugsweise genannt:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)-benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Es ist auch besonders vorteilhaft, die erfindungsgemäßen Wirkstoffkombinationen mit Salicylsäurederivaten zu kombinieren, von denen einige Vertreter bekannt sind, welche ebenfalls UV-Strahlung absorbieren können. Zu gebräuchlichen UV-Filtern gehören

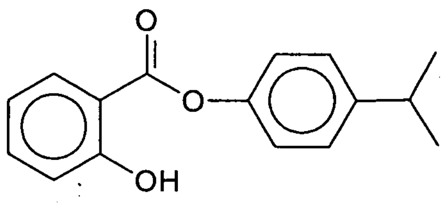

(4-Isopropylbenzylsalicylat),

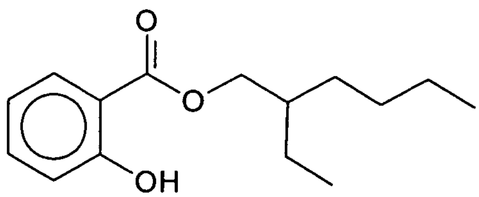

(2-Ethylhexylsalicylat, Octylsalicylat),

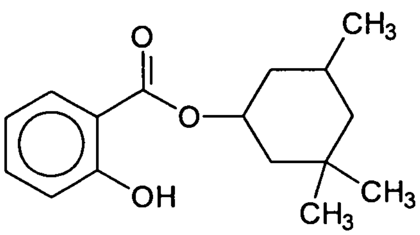

(Homomenthylsalicylat).

Die Herstellung der erfindungsgemäßen kosmetischen und/oder dermatologischen Zubereitungen erfolgt in der dem Fachmann üblichen Weise, zumeist dergestallt, dass eine Ölphase mit einem Gehalt an erfindungsgemäß verwendeten grenzflächenaktiven Glucosederivaten bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wässrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen lässt. Nach Abkühlen auf Raumtemperatur kann, insbesondere, wenn noch flüchtige Bestandteile eingearbeitet werden sollen, nochmaliges Homogenisieren erfolgen.

Dem Fachmann ist natürlich bewusst, dass die Verwendung von Elektrolyten die Leistung von Verdickern und Gelbildnern beeinflussen kann. Diese werden eingesetzt, um die rheologischen und sensorischen Eigenschaften eines Produktes zu optimieren. Durch die hohe Ionenstärke von erfindungsgemäßen Zubereitungen kann die Einstellung einer hohen Viskosität erschwert werden, weswegen es vorteilhaft ist, so genannte "elektrolytstabile Verdicker" einzusetzen. Unter "elektrolytstabilen Verdickern" sind polymere Hydrokolloide zu verstehen wie beispielsweise pflanzliche Gumme, Polysaccharide und Cellulosen, Schichtsilikate, Polytaurate, Polyacrylate und Polyacrylamide, Derivate der vorgenannten Stoffe sowie Kombinationen der vorgenannten Stoffe und/oder ihrer Derivate.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Vorteilhaft sind außerdem Taurate, z.B. Ammonium Acryloyldimethyltaurat / VP Copolymer oder Polyacryldimethyltauramid.

Unter den Polyacrylaten befinden sich z.B. Carbopol Typen der Firma Goodrich (Carbopol 980, 981, 1382, 5984, 2984, ETD 2001, ETD 2020, ETD 2050, Ultrez-10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**O/W-Emulsionen:**

| **Beispiel 1** | **Gew.-%** |
|---|---|
| Glycerylstearat | 3,0 |
| PEG-40-Stearat | 1,0 |
| Cetearylalkohol | 3,0 |
| Cera Microcristallina | 1,0 |
| Capryl-/Caprinyltriglyceride | 3,0 |
| Sheabutter | 1,0 |
| $C_{12-15}$-Alkylbenzoat | 3,0 |
| Macadamiaöl | 1,0 |
| Dimethicon | 1,0 |
| Butylmethoxydibenzoylmethan | 2,0 |
| Ethylhexylmethoxycinnamat | 3,0 |
| Harnstoff | 1,0 |
| Tocopherylacetat | 1,0 |
| Glycerin | 10,0 |
| Butylenglykol | 1,0 |
| Ethylhexylglycerin | 0,5 |
| NA-EDTA | 0,2 |
| Phenylbenzimidazol-5-sulfonsäure | 2,0 |
| Distärkephosphat | 1,0 |
| Carbomer | 0,3 |
| Kreatinphosphat | 1,5 |

(fortgesetzt)

| Beispiel 1 | Gew.-% |
|---|---|
| Kreatinin | 0,3 |
| NaCl | 0,4 |
| Niacinamid | 0,2 |
| Xanthan Gum | 0,2 |
| Mica | 1,0 |
| Parabene | 0,8 |
| Iodopropinylbutylcarbamat | 0,05 |
| NaOH | q.s. |
| Citronensäure | q.s. |
| Parfum | q.s. |
| entsalztes Wasser | ad 100 |

| Beispiel 2 | Gew:% |
|---|---|
| Glycerylstearat | 3,0 |
| PEG-40-Stearat | 1,0 |
| Cetearylalkohol | 2,0 |
| gehärtete Kokosglyceride | 1,0 |
| Shea Butter | 2,0 |
| $C_{12-15}$-Alkylbenzoat | 2,0 |
| Bienenwachs | 1,0 |
| Cyclomethicon | 2,0 |
| Ethylhexylmethoxycinnamat | 5,0 |
| Benzophenon-3 | 3,0 |
| Phenylbenzimidazol-5-sulfonsäure | 1,5 |
| Ubichinon | 0,05 |
| Glycerin | 7,0 |
| Tocopherylacetat | 0,5 |
| Na-Iminodisuccinat | 0,2 |
| Methylpropandiol | 1,0 |
| Carrageenan | 0,2 |
| Tapiokastärke | 0,5 |
| Carbomer | 0,4 |
| Kreatinin | 0,5 |
| Kreatin | 1,0 |
| Natriumascorbylphosphat | 0,1 |
| Panthenol | 1,0 |
| Parabene | 0,4 |

(fortgesetzt)

| Beispiel 2 | Gew:% |
|---|---|
| Iodopropinylbutylcarbamat | 0,05 |
| NaOH | q.s. |
| Parfum | q.s. |
| entsalztes Wasser | ad 100 |

| Beispiel 3 | Gew.% |
|---|---|
| Glycerylstearatcitrat | 2,5 |
| Cetylalkohol | 3,0 |
| Stearylalkohol | 2, |
| Capryl-/Caprinyltriglyceride | 1,0 |
| Sheabutter | 1,5 |
| gehärtete Kokosglyceride | 1,0 |
| Cyclomethicone | 2,0 |
| Ethylhexylcocoat | 1,0 |
| Dipentaerythritylhexacaprylat/-hexacaprat | 0,5 |
| Dimethicon | 1,0 |
| Kokosglyceride | 2,0 |
| Ethylhexyltriazone | 2,0 |
| Ubichinon | 0,05 |
| Retinylpalmitat | 0,1 |
| Glycerin | 7,0 |
| Panthenol | 1,0 |
| EDTA | 0,2 |
| Phenylen-1,4-bis-(2-benzimidazyl)-3,3'-5,5'-tetrasulfonsäure | 2,0 |
| $TiO_2$ | 1,0 |
| Xanthangummi | 0,2 |
| Carbomer | 0,3 |
| Kreatin | 2,0 |
| Butylenglycol | 3,0 |
| Parabene | 0,5 |
| Iodopropinylbutylcarbamat | 0,05 |
| NaOH | q.s. |
| Citronensäure | q.s. |
| Parfum | q.s. |
| entsalztes Wasser | ad 100 |

| Beispiel 4 | Gew.% |
|---|---|
| Glycerylstearatcitrat | 2 |
| Myristylmyristat | 1 |
| Stearylalkohol | 2 |
| Cetylalkohol | 1 |
| Hydrierte Kokosfettglyceride (Hydrogenated Coco Glycerides) | 2 |
| Butylenglycol Dicaprylat/Dicaprat | 1 |
| Ethylhexylkokosfettsäureester | 3 |
| Isohexadecan | 2 |
| Vaseline | 1 |
| Dicarprylylcarbonat | 2 |
| $TiO_2$ | 1 |
| Ethylhexylmethoxycinnamat | 5 |
| Butylmethoxydibenzoylmethan | 2 |
| Ethylhexylsalicylat | 1 |
| Niacinamid | 0,3 |
| Tocopherylacetat | 1 |
| Kreatin | 1,0 |
| Kreatinin | 0,2 |
| Phenoxyethanol | 0,6 |
| p-Hydroxybenzoesäurealkylester (Paraben) | 0,2 |
| Diazolidinylharnstoff | 0,25 |
| Polyacrylsäure (Carbomer) | 0,4 |
| Carageenan | 0,2 |
| Glycerin | 8,0 |
| wasser- und/oder öllösliche Farbstoffe | 0,05 |
| Natriumlactat | 1,0 |
| Natriumglycinat | 0,5 |
| Additive (Distärkephosphat, EDTA, BHT, Talkum) | 0,5 |
| Parfum | q.s. |
| entsalztes Wasser | ad 100 |

**W/O/W-Emulsion**

| Beispiel 5 | Gew.% |
|---|---|
| PEG-40-Stearat | 1,5 |
| Glycerylstearat | 2,5 |
| Cetylalkohol | 3,0 |
| Octamethyltetrasiloxan (Cyclomethicon) | 2,0 |
| Ricinusöl | 4,0 |

(fortgesetzt)

| Beispiel 5 | Gew.% |
|---|---|
| Isopropylpalmitat | 4,5 |
| Kreatin | 0.5 |
| Sericoside | 0,2 |
| Na-Ascorbylphosphat | 0,1 |
| $C_{12-15}$ Alkyl Benzoat | 2,0 |
| Glycerin | 7,0 |
| Butylmethoxydibenzoylmethan | 1,0 |
| Ethylhexylmethoxycinnamat | 2,0 |
| Phenoxyethanol | 0.3 |
| 1,3-Dimethylol-5,5-dimethyl-hydantoin (DMDM Hydantoin) | 0.1 |
| lodopropinylbutylcarbamat | 0.05 |
| Magnesiumchlorid | 0,5 |
| Magnesiumsulfat | 1 |
| Zitronensäure, Natriumsalz | 0.2 |
| Trinatrium EDTA | 0.2 |
| Parfum | q.s. |
| entsalztes Wasser | ad 100 |

Die Fettphase, welche den Emulgator enthält, wird auf 80°C erhitzt, getrennt davon ebenso die Wasserphase. Bei 80°C werden beide Phasen miteinander vereinigt, ca. 10 Minuten lang homogenisiert und anschließend auf Raumtemperatur abgekühlt (Eintopfverfahren).

**W/O-Emulsionen**

| Beispiel 6 | Gew.-% |
|---|---|
| Polyglyceryl-3 Diisostearat | 1,5 |
| PEG-40 Sorbitan Perisostearat | 2,5 |
| Lanolin Alcohol | 0,3 |
| Mineral Oil | 8,0 |
| Cera Microcrystallina | 2,5 |
| Octamethyltetrasiloxan (Cyclomethicon) | 4,0 |
| Isohexadecan | 2,0 |
| Isopropylpalmitat | 5,0 |
| Kreatin | 0.5 |
| Kreatinin | 0,1 |
| Tocopherylacetat | 1.0 |
| lodopropinylbutylcarbamat | 0,1 |
| Magnesiumsulfat | 0,6 |
| Milchsäure | 0,7 |
| Milchsäure, Natriumsalz | 1,5 |

(fortgesetzt)

| Beispiel 6 | Gew.-% |
|---|---|
| Glycerin | 7,0% |
| Parfum | q.s. |
| entsalztes Wasser | ad 100% |

| Beispiel 7 | Gew.-% |
|---|---|
| Glycerylsterat | 1 |
| Stearinsäure | 3 |
| Behenylalkohol | 2 |
| Cetylalkohol | 2 |
| Hydrierte Kokosfettglyceride (Hydrogenated Coco Glycerides) | 1 |
| $C_{12-15}$ Alkylbenzoat | 2 |
| Octyldodecanol | 2 |
| Octamethyltetrasiloxan (Cyclomethicon) | 2 |
| Dimethylpolysiloxan (Dimethicon) | 1 |
| Dicarprylylcarbonat | 4 |
| Butylmethoxydibenzoylmethan | 1 |
| Ethylhexylmethoxycinnamat | 2 |
| Phenylbenzimidazol-sulfonsäure | 0,5 |
| Ubichinon (Q10) | 0,05 |
| Kreatin | 0,1 |
| Kreatinin | |
| Natriumascorbylphosphat | 0,3 |
| Tocopherylacetat | 0,5 |
| Iminodisuccinat | 0,2 |
| Phenoxyethanol | 0,3 |
| p-Hydroxybenzoesäurealkylester (Paraben) | 0,6 |
| Ethanol denaturiert | 3 |
| Carrageenan | 0,2 |
| Polyacrylsäure (Carbomer) | 0,2 |
| Glycerin | 7 |
| Additive (Mica, Citronensäure, BHT) | 0,5 |
| Parfüm | q.s. |
| Wasser | ad 100 |

| Beispiel 8 | Gew.% |
|---|---|
| Cetearylglucosid | 2 |
| Myristylmyristat | 1 |

(fortgesetzt)

| Beispiel 8 | Gew.% |
|---|---|
| Stearylalkohol | 4 |
| C12-15 Alkylbenzoat | 3 |
| Caprylsäure/Caprinsäure Triglycerid | 3 |
| Hydriertes Polydecen | 1 |
| Dicarprylylcarbonat | 3 |
| Polydecen | 2 |
| Ethylhexylmethoxycinnamat | 3 |
| Ethylhexylcyanodiphenyl-acrylat (Octocrylen) | 3 |
| Phenylen-1,4-bis-(2-benzimidazyl)-3,3'-5,5'-tetrasulfonsäure | 0,5 |
| Kreatin | 1,5 |
| Ubichinon (Q10) | 0,1 |
| Tocopherylacetat | 0,5 |
| Trinatrium EDTA | 0,2 |
| Phenoxyethanol | 0,4 |
| p-Hydroxybenzoesäurealkylester (Paraben) | 0,3 |
| Ethylhexylglycerin | 0,4 |
| Ammoniumpolyacryloyldimethyltaurate | 0,3 |
| Aluminium Stärke Octenylsuccinate | 1 |
| Glycerin | 4 |
| Butylenglycol | 2 |
| Additive (Milchsäure, BHT) | 0,8 |
| Parfüm | q.s. |
| Wasser | ad 100 |

| Beispiel 9 | Ges.-% |
|---|---|
| PEG-30 Dipolyhydroxystearat | 3,0 |
| Caprylic/Capric Triglycerid | 5 |
| Mineral Oil | 12,5 |
| Kreatin | 0.3 |
| Vitamin K | 0,1 |
| Na-Ascorbylphosphat | 0.3 |
| Phenoxyethanol | 0,5 |
| Butylmethoxydibenzoylmethan | 1,0 |
| Ethylhexylmethoxycinnamat | 3,0 |
| Milchsäure | 0,7 |
| Milchsäure, Natriumsalz | 1,5 |
| Glycerin | 8,0 |

(fortgesetzt)

| Beispiel 9 | Ges.-% |
|---|---|
| Parfum | q.s. |
| entsalztes Wasser | ad 100 |

| Beispiel 10 | Gew.-% |
|---|---|
| Cetyl Dimethicon Copolyol | 2,0 |
| Octamethyltetrasiloxan (Cyclomethicon) | 5,0 |
| Dimethylpolysiloxan (Dimethicon) | 5,0 |
| Phenyltrimethicon | 3,0 |
| Isohexadecan | 3,0 |
| Dimethiconol | 1,0 |
| Phenoxyethanol | 0,5 |
| p-Hydroxybenzoesäurealkylester (Paraben) | 0.2 |
| Iodopropinylbutylcarbamat | 0,05 |
| Natriumchlorid | 2,0 |
| Kreatin | 0,5 |
| Allantoin | 0,5 |
| Na-Ascorbylphosphat | 0.1 |
| Butylenglycol | 3,0 |
| Glycerin | 6,0 |
| Parfum | q.s. |
| entsalztes Wasser | ad 100 |

**Patentansprüche**

**1.** Kosmetische Zubereitung mit einer wässrigen Phase, enthaltend

(a) Kreatin und/oder Kreatinin
(b) wobei in der der wässrige Phase ein oder mehrere Elektrolyte gewählt aus der Gruppe

(1) der Salze mit folgenden Anionen: Chloride, ferner anorganische Oxo-Element-Anionen, von diesen insbesondere Sulfate, Carbonate, Phosphate, Borate und Aluminate, auf organischen Anionen basierende Elektrolyte, z.B. Lactate, Acetate, Benzoate, Propionate, Tartrate, Citrate, Aminosäuren, Ethylendiamintetraessigsäure und deren Salze
und folgenden Kationen: Ammonium,- Eisen-, Kupfer-, Zink-, Aluminium-, Alkylammonium,- Alkalimetall-, Erdalkalimetall,- insbesondere Natrium-, Calcium- und Magnesiumionen;
(2) der als Alkalisalze vorliegenden wasserlöslichen UV-Filtersubstanzen, insbesondere solcher, die an ihrem Molekülgerüst eine oder mehrere Sulfonsäuregruppen bzw. Sulfonatgruppen tragen;
(3) der Aminosäuren und deren Salze bzw. deren Anionen;
(4) der kosmetisch und dermatologisch relevanten $\alpha$-Hydroxycarbonsäuren, $\alpha$-Ketocarbonsäuren und $\beta$-Hydroxycarbonsäuren und insbesondere deren Salze, wobei die Kationen vorteilhaft gewählt werden können aus der Gruppe Ammonium,-Alkylammonium,-Alkalimetall-, Erdalkalimetall,- Magnesium-, Eisen- bzw. Zinkionen
gelöst enthalten sind, wobei die Ionenstärke der wässrigen Phase, in der der oder die Elektrolyte gelöst

vorliegen, mindestens 0,050 mol/l beträgt und mindestens diese Wasserphase einen pH-Wert von 6,2 bis 7,8, besonders bevorzugt 6,5 bis 7,5 aufweist.

2. Zubereitung nach Anspruch 2, **dadurch gekennzeichnet, dass** der oder die Elektrolyte aus der Gruppe der als Alkalisalze vorliegenden wasserlöslichen UV-Filtersubstanzen gewählt wird aus der Gruppe der 2-Phenylbenzimidazol-5-sulfohsäure und deren Salzen, der Sulfonsäure-Derivate von Benzophenonen und deren Salzen, der Sulfonsäure-Derivate des 3-Benzylidencamphers und deren Salzen, der 2-Methyl-5-(2-oxo-3-bornylidenmethyl)benzolsulfonsäure und deren Salzen, des 1,4-di(2-oxo-10-Sulfo-3-bornylidenmethyl-)Benzols und dessen Salzen sowie der Phenylen-1,4-bis-(2-benzimidazyl)-3,3'-5,5'-tetrasulfonsäure und deren Salzen.

3. Zubereitung nach einem der vorstehenden Ansprüche, **dadurch gekennzeichnet, dass** es sich um eine Emulsion oder ein Hydrogel handelt.

4. Zubereitung nach einem vorstehenden Ansprüche, **dadurch gekennzeichnet, dass** sie ein polymeres Hydrokolloid enthält.

5. Zubereitung nach Anspruch 5, **dadurch gekennzeichnet, dass** das polymere Hydrokolloid gewählt ist aus der Gruppe bestehend aus wasserlöslichen Polyacrylsäuren oder Polymethacrylsäure und ihren Salzen und Derivaten, wasserlöslichen Copolymeren und/oder Blockcopolymeren von Acrylsäure mit Alkylacrylsäure, Polyacrylamiden und ihren Derivaten, wasserlöslichen Polysacchariden wie Cellulose und Gellulosederivaten, Gummen und ihren Derivaten wie zum Beispiel Xanthan Gum, Carrageenan, sowie aus Tauraten und ihren Derivaten wie Polyacryldimethyltauramid und Ammoniumacrylolyfdimethyltaurat/Vinylpyrrolidon-Copolymer.

6. Zubereitung nach einem der vorstehenden Ansprüche, **dadurch gekennzeichnet, dass** es sich um eine O/W- oder W/O/W-Emulsion handelt, die einen elektrolytstabilen Emulgator enthält.

7. Zubereitung nach Anspruch 6, **dadurch gekennzeichnet, dass** der elektrolytstabile Emulgator Polyethylenglykol- oder Polyglycerylketten enthält.

8. Zubereitung nach Anspruch 7, **dadurch gekennzeichnet, dass** der elektrolytstabile Emulgator PEG-100-Stearat, PEG-40-Stearat, PEG-30-Stearat, PEG-25-Stearat, PEG-20-Glycerylstearat, PEG-25 Hydriertes Ricinusöl, Ceteareth-25, Ceteareth-20, Cetheareth-12, Steareth-21, Steareth-12, Steareth-2, Polyglyceryl-3-Methylglucosedistearat oder Mischungen davon umfasst.

9. Zubereitung nach einem der vorstehenden Ansprüche, enthaltend Kreatin und Kreatinin, wobei das Gewichtsverhältnis von Kreatinin zu Kreatin aus dem Bereich von 10 : 1 bis 1 : 10, besonders bevorzugt von 4 : 1 bis 3 zu 7, ganz besonders bevorzugt 2 : 1 bis 1 : 2 gewählt wird.

## Claims

1. Cosmetic preparation having an aqueous phase, containing

(a) creatine and/or creatinine
(b) wherein the the aqueous phase contains one or more electrolytes selected from the group

(1) of salts having the following anions: chlorides, furthermore inorganic oxo-element anions, of these in particular sulphates, carbonates, phosphates, borates and aluminates, electrolytes based on organic anions, for example lactates, acetates, benzoates, propionates, tartrates, citrates, amino acids, ethylenediaminetetraacetic acid and the salts thereof
and the following cations: ammonium, iron, copper, zinc, aluminium, alkylammonium, alkali metal, alkaline earth metal, in particular sodium, calcium and magnesium, ions;
(2) of water-soluble UV filters present as alkali metal salts, in particular those bearing one or more sulphonic acid groups or sulphonate groups on their molecular backbone;
(3) of amino acids and the salts thereof or the anions thereof;
(4) of cosmetically and dermatologically relevant $\alpha$-hydroxycarboxylic acids, $\alpha$-ketocarboxylic acids and $\beta$-hydroxycarboxylic acids and in particular the salts thereof, wherein the cations can be advantageously selected from the group of ammonium, alkylammonium, alkali metal, alkaline earth metal, magnesium, iron

and zinc ions
in a dissolved state, wherein the ionic strength of the aqueous phase in which the electrolyte(s) is/are present in a dissolved state is at least 0.050 mol/l and at least said aqueous phase has a pH of 6.2 to 7.8, particularly preferably 6.5 to 7.5.

**2.** Preparation according to Claim 2, **characterized in that** the electrolyte(s) from the group of water-soluble UV filters present as alkali metal salts is/are selected from the group of 2-phenylbenzimidazole-5-sulphonic acid and the salts thereof, of sulphonic acid derivatives of benzophenones and the salts thereof, of sulphonic acid derivatives of 3-benzylidene camphor and the salts thereof, of 2-methyl-5-(2-oxo-3-bornylidenemethyl)benzenesulphonic acid and the salts thereof, of 1,4-di(2-oxo-10-sulpho-3-bornylidenemethyl)benzene and the salts thereof and also of phenylene-1,4-bis(2-benzimidazyl)-3,3'-5,5'-tetrasulphonic acid and the salts thereof.

**3.** Preparation according to either of the preceding claims, **characterized in that** it is an emulsion or a hydrogel.

**4.** Preparation according to any of the preceding claims, **characterized in that** it contains a polymeric hydrocolloid.

**5.** Preparation according to Claim 5, **characterized in that** the polymeric hydrocolloid is selected from the group consisting of water-soluble polyacrylic acids or polymethacrylic acid and their salts and derivatives, water-soluble copolymers and/or block copolymers of acrylic acid with alkylacrylic acid, polyacrylamides and their derivatives, water-soluble polysaccharides such as cellulose and cellulose derivatives, gums and their derivatives such as, for example, xanthan gum, carrageenan, and also of taurates and their derivatives such as polyacryldimethyltauramide and ammonium acryloyldimethyltaurate/vinylpyrrolidone copolymer.

**6.** Preparation according to any of the preceding claims, **characterized in that** it is an O/W or W/O/W emulsion containing an electrolyte-stable emulsifier.

**7.** Preparation according to Claim 6, **characterized in that** the electrolyte-stable emulsifier contains polyethylene glycol chains or polyglyceryl chains.

**8.** Preparation according to Claim 7, **characterized in that** the electrolyte-stable emulsifier comprises PEG-100 stearate, PEG-40 stearate, PEG-30 stearate, PEG-25 stearate, PEG-20 glyceryl stearate, PEG-25 hydrogenated castor oil, ceteareth-25, ceteareth-20, ceteareth-12, steareth-21, steareth-12, steareth-2, polyglyceryl-3 methylglucose distearate or mixtures thereof.

**9.** Preparation according to any of the preceding claims, containing creatine and creatinine, wherein the weight ratio of creatinine to creatine is selected from the range of 10:1 to 1:10, particularly preferably of 4:1 to 3:7, very particularly preferably 2:1 to 1:2.

## Revendications

**1.** Préparation cosmétique présentant une phase aqueuse, contenant

(a) de la créatine et/ou de la créatinine
(b) un ou plusieurs électrolytes, choisis dans le groupe

(1) des sels présentant les anions suivants : les chlorures, en outre les anions d'oxo-éléments inorganiques, parmi lesquels en particulier les sulfates, les carbonates, les phosphates, les borates et les aluminates, en outre des électrolytes à base d'anions organiques, par exemple les lactates, les acétates, les benzoates, les propionates, les tartrates, les citrates, les acides aminés, l'acide éthylènediaminetétraacétique et leurs sels.
et les cations suivants : les ions d'ammonium, de fer, de cuivre, de zinc, d'aluminium, d'alkylammonium, de métal alcalin, de métal alcalino-terreux, en particulier de sodium, de calcium et de magnésium ;
(2) des substances filtre des UV, solubles dans l'eau, se trouvant sous forme de sels de métal alcalin, en particulier celles qui présentent un ou plusieurs groupes acide sulfonique ou sulfonate sur leur structure moléculaire ;
(3) des acides aminés et de leurs sels ou leurs anions ;
(4) des acides $\alpha$-hydroxycarboxyliques, $\alpha$-cétocarboxyliques et ß-hydroxycarboxyliques cosmétiquement

et dermatologiquement importants et en particulier de leurs sels, les cations pouvant avantageusement être choisis dans le groupe des ions d'ammonium, d'alkylammonium, de métal alcalin, de métal alcalino-terreux, de magnésium, de fer ou de zinc,
contenus sous forme dissoute dans la la phase aqueuse, la force ionique de la phase aqueuse, dans laquelle le ou les électrolytes se trouvent sous forme dissoute, valant au moins 0,050 mole/l et au moins cette phase aqueuse présentant un pH de 6,2 à 7,8, de manière particulièrement préférée de 6,5 à 7,5.

**2.** Composition selon la revendication 2, **caractérisée en ce que** le ou les électrolytes du groupe des substances filtre des UV solubles dans l'eau, se trouvant sous forme de sels de métal alcalin, sont choisis dans le groupe formé par l'acide 2-phénylbenzimidazole-5-sulfonique et ses sels, les dérivés de l'acide sulfonique de benzophénones et leurs sels, les dérivés de l'acide sulfonique du 3-benzylidènecamphre et leurs sels, l'acide 2-méthyl-5-(2-oxo-3-bornylidèneméthyl)benzènesulfonique et ses sels, le 1,4-di(2-oxo-10-sulfo-3-bornylidèneméthyl)benzène et ses sels ainsi que l'acide phénylène-1,4-bis-(2-benzimidazyl)-3,3'-5,5'-tétrasulfonique et ses sels.

**3.** Préparation selon l'une quelconque des revendications précédentes, **caractérisée en ce qu'**il s'agit d'une émulsion ou d'un hydrogel.

**4.** Préparation selon l'une quelconque des revendications précédentes, **caractérisée en ce qu'**elle contient un hydrocolloïde polymère.

**5.** Préparation selon la revendication 5, **caractérisée en ce que** l'hydrocolloïde polymère est choisi dans le groupe constitué par les poly(acides acryliques) ou le poly(acide méthacrylique) solubles dans l'eau et leurs sels et dérivés, les copolymères et/ou les copolymères séquencés, solubles dans l'eau, de l'acide acrylique avec de l'acide alkylacrylique, les polyacrylamides et leurs dérivés, les polysaccharides solubles dans l'eau, tels que la cellulose et les dérivés de cellulose, les gommes et leurs dérivés, tels que par exemple la gomme de xanthane, le carraghénane, ainsi que les taurates et leurs dérivés, tels que le polyacryldiméthyltauramide et le copolymère d'acryloyldiméthyltaurate d'ammonium/vinylpyrrolidone.

**6.** Préparation selon l'une quelconque des revendications précédentes, **caractérisée en ce qu'**il s'agit d'une émulsion H/E ou E/H/E, qui contient un émulsifiant stable aux électrolytes.

**7.** Préparation selon la revendication 6, **caractérisée en ce que** l'émulsifiant stable aux électrolytes contient des chaînes polyéthylèneglycol ou polyglycéryle.

**8.** Préparation selon la revendication 7, **caractérisée en ce que** l'émulsifiant stable aux électrolytes comprend le stéarate de PEG-100, le stéarate de PEG-40, le stéarate de PEG-30, le stéarate de PEG-25, le polyglycérylstéarate de PEG-20, l'huile de ricin hydrogénée par PEG-25, le cétéareth-25, le cétéareth-20, le cétéareth-12, le stéareth-21, le stéareth-12, le stéareth-2, le 3-methylglucosedistéarate de polyglycéryle ou des mélanges de ceux-ci.

**9.** Préparation selon l'une quelconque des revendications précédentes, contenant de la créatine et de la créatinine, le rapport pondéral de créatinine à créatine étant choisi dans la plage de 10:1 à 1:10, de manière particulièrement préférée de 4:1 à 3:7, de manière tout particulièrement préférée de 2:1 à 1:2.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10032964
- JP 2000247866 A
- WO 0033787 A
- EP 565010 A
- US 4590067 A
- EP 178602 A
- DE 3314742 A

### In der Beschreibung aufgeführte Nicht-Patentliteratur

- *Int. J. Cosm. Science,* 1988, vol. 10, 53